# EUROPEAN PATENT SPECIFICATION

(11) **EP 0 481 704 B1**
(45) Date of publication and mention of the grant of the patent: **25.09.1996**
(21) Application number: 91309430.6
(22) Date of filing: 14.10.1991
(51) Int. Cl.: C12Q 1/68

(54) **Amplification of midivariant DNA templates**
Amplifikation von Midivariant-DNS-Templaten
L'amplification de matrices d'ADN de midivariant

(30) Priority: 16.10.1990 US 598269
(43) Date of publication of application: 22.04.1992
(60) Divisional application: 95202169.9
(73) Proprietor: Ciba Corning Diagnostics Corp., Medfield Massachusetts 02052 (US)
(72) Inventor: Martinelli, Richard M., Brighton, MA 02135 (US); Donahue, Jeffrey J., Wayland, MA 01778 (US); Unger, John T., Medfield, MA 02052 (US)
(74) Representative: Froud, Clive

(56) References cited:
- EP-A- 0 361 983
- EP-A- 0 379 369
- WO-A-90/02820
- WO-A-90/03446
- WO-A-90/06376
- WO-A-90/14439

## Description

The present invention generally relates to amplification methods and more particularly, amplification including midivariant DNA/probe conjugates.

Nucleic acid based test assays provide distinct advantages in specificity and sensitivity over conventional immunoassay procedures. The essential feature of nucleic acid based test assays is the hybridization of target specific nucleic acid sequences (probes) to their complementary target nucleic acid sequences in a test sample. A probe's specificity relates to its ability to distinguish between target and non-target nucleic acid sequences. Probe specificity may be absolute (i.e. probe able to distinguish between target nucleic acid sequences and non-target nucleic acid sequences), or it may be functional (i.e. probe able to distinguish between the target nucleic acid sequence and any other nucleic acid sequence normally present in a test sample). A target nucleic acid sequence of a test sample in a test assay refers to a segment of single-stranded polynucleotide having a nucleotide base sequence corresponding to a genetic element whose presence in a test sample is to be determined. The test sample refers to any test sample containing one or more target nucleic acids and which may be in purified or nonpurified form. Sources for test sample may include, but should not be limited to, physiological and nonphysiological DNA or RNA (synthesized or natural), and the like.

It has been noted that the whole repertoire of antigenic determinants of two separate but related organisms may not permit their discrimination by immunoassay, whereas unique genomic sequences can be identified and differentiated by a nucleic acid test assay. See U.S. Patent 4,851,336. Also, in the case of viral targets, i.e. HIV infection, antibodies to viral antigens may not be detectable even though viral sequences have been inserted into the host genome, however, the insertion will provide a potential diagnostic marker if a detection (amplification) methodology of the requisite sensitivity can be devised. The present invention provides amplification methodology(s) of the requisite sensitivity.

It is now realized that the full capability, in terms of sensitivity, of nucleic acid based test assays can only be achieved in conjunction with a method for the amplification of a detectable molecule indicative of the presence of the target nucleic acid sequence in the test sample.

The polymerase chain reaction (PCR) is one type of amplification method where the target sequence is first amplified and then isolated. Since PCR amplification occurs early in the process, sequences other than the target may also amplify. Identification of the actual target requires the analysis of the amplified sequences. See U.S. Patents 4,683,195 and 4,683,202.

U.S. Patents 4,786,660 and 4,957,858 describe an autocatalytic replication of recombinant RNA by QB replicase, another type of amplification method where the target sequence is first isolated and then amplified. The disadvantage of this method of QB amplification is that probes not hybridized to the target nucleic acid sequence must be made vulnerable to destruction i.e. rendered unreplicable. See "Amplifying Probe Assays with Q-Beta Replicase" Bio/Technology 1989: 7(6), 609-10 (Eng.) and "Q Beta Amplification" J. Clin. Lab. Anal. 4:318 (1990) (letter).

WO-A-9014439 describes a method for detecting a nucleic acid target sequence with a replicable RNA reporter system. The method provides two probe-primers which function in target-specific hybridization and in priming DNA polymerization in a complementary DNA synthesis reaction, each probe is utilized in a separate polymerase-mediated reaction that generates a target-specific gene containing a DNA sequence that then serves as a template for the synthesis of a replicable RNA. More specifically, a first probe-primer is hybridized to the first region of a target sequence and is then extended on the template of the target sequence through at least the second region of the target sequence. The resulting extension product is then separated from the target sequence. The second probe-primer is then hybridized to the extension product and extended on the template of the extension product through the promoter sequence. The second extension results in the creation of a double-stranded DNA with a functional double-stranded promoter that can direct the transcription of the gene thereby generating a replicable RNA.

WO-A-9006376 describes a target nucleic acid amplification/detection system which utilizes nucleic acid sequences, one having a probe sequence linked to a sequence capable of initiating replication by an RNA-dependent RNA polymerase, and another reporter molecule capable of hybridizing to a strand separated from the extension product of the first nucleic acid sequence after hybridization to a specific target sequence. The extension product of the second hybridized nucleic tied sequence then serves as a template source for autocatic replication by an RNA-dependent RNA polymerase.

Each of the above applications employs two cycles of hybridization of a primer-probe, followed by extension of the probe. In addition, the target complementary strands are complementary to different strands of the target sequences, as a result of the first extension reaction.

An amplification method(s) is described and includes as a first step the hybridization of two distinct nonreplicable midivariant DNA/probe conjugates to a target nucleic acid sequence in a test sample. The test sample being previously treated, if necessary, to release the target nucleic acid sequences. These conjugates each include at least two parts, one part consists of a target specific nucleic acid sequence (probe), which is complementary to that of the target nucleic acid sequence, under hybridization conditions, and the other part being a nonreplicable portion of the midivariant DNA. One conjugate will contain a portion of the 5′ end of one strand of the midivariant DNA and the other conjugate will contain the remaining nonreplicable portion of the midivariant DNA to its 3′ terminus. The probes of the conjugates are chosen so that upon hybridization to the target molecule the two probes are aligned contiguous to each other. In the next step the probes are covalently coupled by T4 DNA ligase to generate a substrate for replication. The enzyme QB replicase uses the midivariant DNA as a template to produce copies of the RNA midivariant sequence with the target specific sequence inserted. The products of the replication are detected and are indicative of the presence of target molecule in the test sample.

In a modified amplification method one of the portions of the nonreplicable midivariant DNA of one conjugate includes an attached RNA polymerase promoter. The RNA polymerase promoter operates as a double stranded molecule. Following hybridization and ligation the DNA template is transcribed into RNA. The RNA transcript is then replicated by QB replicase, and the replication product detected.

A conjugate and a pair of conjugates are described for use in the amplification method(s) of the present invention.

The midivariant DNA/probe conjugates and other components as described herein are adapted to be packaged in a test kit for performing the amplification method(s).

It is a primary objective of the invention to provide an amplification method incorporating midivariant DNA.

It is still another object of the invention to provide a conjugate including a nonreplicable portion of midivariant DNA and a target specific nucleic acid sequence.

A further objective of the invention is to provide two distinct conjugates, each including, a distinct nonreplicable portion of midivariant DNA and a distinct target specific nucleic acid sequence, and where upon hybridization of both target specific nucleic acid sequences to a target nucleic acid sequence in a test sample, the probes are ligated and the portions of the midivariant DNA then serve as a template for replication, with the products of the replication being detectable.

Another object of the invention is to provide an amplification method, including hybridization, ligation and replication steps that permit the detection of less than 200 target molecules in a test sample.

A still further object of the invention is to provide a modified amplification method including hybridization, ligation, transcription and replication steps that permit the detection of one target molecule in a test sample.

It is still another objective of the invention to provide a test kit for performing an amplification method in order to detect replication products indicative of the presence of a target nucleic acid sequence in a test sample.

Another objective of the invention is to provide an amplification method which affords significant advantage of sensitivity compared to immunoassays.

Referring to the accompanying illustrative drawings:

FIG. 1 is a representative illustration of replication catalyzed by QB replicase.

FIG. 2 is a representative illustration of the hybridization-ligation-replication method of the present invention.

FIG. 3 is a representative illustration of the hybridization-ligation-transcription-replication method of the present invention. FIG. 4 is a partial restriction map of pMDV.

FIG. 5 illustrates the results of gel electrophoresis of products of the replication of the present invention.

FIG. 6 illustrates the results of gel electrophoresis for the hybridization-ligation-replication reaction of Example 3.

FIG. 7 illustrates the results of the transcription-replication reaction with MDV-CA83 of Example 4.

FIG.8 illustrates the results of the transcription-replication reaction with MDV-SH of Example 4.

FIG. 9 illustrates the results of the transcription-replication reaction with MDV-SA2 of Example 4.

In the examples provided below the product of the replication step of the amplification methods is detected by radioisotopic techniques. In a preferred embodiment, however, the products of the replication step of the amplification are detected by chemiluminescent methodology. See U.S. Patent 4,745,181. An immunoassay format representative of this methodology in the case of probe technology, provides that a complimentary "reagent" sequence directed toward a target molecule of interest believed to be present in a test sample is is immobilized upon paramagnetic particle (PMP) (Ciba Corning Diagnostics Corp.), a solid phase, and a second complementary "reagent" sequence to the target molecule is labelled with an acridinium ester (Ciba Corning Diagnostics Corp.). Upon incubation of the reagents with the test sample, a sandwich complex is formed with the target molecule. A magnetic field is applied and the PMP are separated out of solution, this effects a separation of the unbound acridinium ester labelled complementary reagent sequence from that which is specifically bound to the analyte on the PMP. After this separation step, the target sequence is detected by the chemiluminescent reaction of labelled complementary reagent sequence which has remained bound to the PMP. In the present invention this chemiluminescent methodology may be applied to detect the replication products. The amplification methodology may also be applied to immunoassays.

Another detection methodology incorporates the use of optical devices to propagate radiation by "total internal reflection" to generate an evanescent wave at the interface of the device and a test medium having a lower index of refraction. See Harrick, N.J., "Internal Reflection Spectroscopy", Harrick Scientific Corp., Ossining N.Y. (Third printing) (1987) and U.S. Patent 4,880,752. The evanescent wave is an electromagnetic waveform which typically extends less than a wavelength into the test medium. However, this penetration is sufficient to permit interaction between the evanescent wave component and the target in the test medium. Alternatively, the target may be attached to the sensor. Applying this methodology to the present invention, analysis of the product of the replication of the amplification method may be achieved by attaching a reactant coating onto an evanescent sensor, from which the product(s) is sensed in the evanescent zone. A reactant coating is defined to include the attachment by coating means of a molecule which is receptive to a complimentary molecule in a test medium to form a binding pair, and the coating process shall include noncovalent binding or covalent binding.

Nucleic acid sequences unique to and indicative for certain pathogens in various test samples have been reported. The source of target nucleic acid sequences in a test sample is defined to include, but not be limited to, cells, viruses and viroids. Although some test samples have been shown by quantitative culture methods to contain as high as 10⁷-10⁹ cells of the infectious organism, in some Salmonella test samples as few as 1000 organisms per gram were shown present.

The current limit of sensitivity of chemiluminescent based detection methodology is approximately 100 amol (60 million molecules). This level of sensitivity was observed for an assay in which a chemiluminescent labelled DNA oligomer was hybridized to Campylobacter 16S ribosomal RNA. Since the required sensitivity of these assays, for example, for infectious disease agents must be as low as 1000 molecules, the target molecules must be amplified by at least 10⁴-10⁵ in order to be detected by chemiluminescent based detection methodology.

A means of obtaining the necessary sensitivity for these test assays involves an amplification method incorporating the use of the enzyme QB replicase. See "Autocatalytic Replication of a Recombinant RNA" J. Mol. Bio. 171:281-95 (1983). The QB virus is an RNA phage whose genome consists of a strand of approximately 4400 ribonucleotides. See Blumenthal, (1982) In "The Enzyme" (Vol. XV, Part B) (P.D. Boyer, ed.) p. 267, Academic Press, New York. The role of QB replicase is to catalyze the synthesis of the complementary strand of RNA from this RNA template. The enzyme consists of 4 subunits, only one of which is actually coded for by the viral genome, the other subunits are derived from the bacterial host. Although QB replicase exhibits narrow specificity in that it will only use QB RNA as a template for complementary strand synthesis for replication (other viral RNA sequences are not replicated), shorter, naturally occurring mutants of QB RNA can also serve as templates for replication. These shorter templates include"nanovariant" RNA which is 90 ribonucleotides in length and "midivariant" RNA which is 220 nucleotides in length.

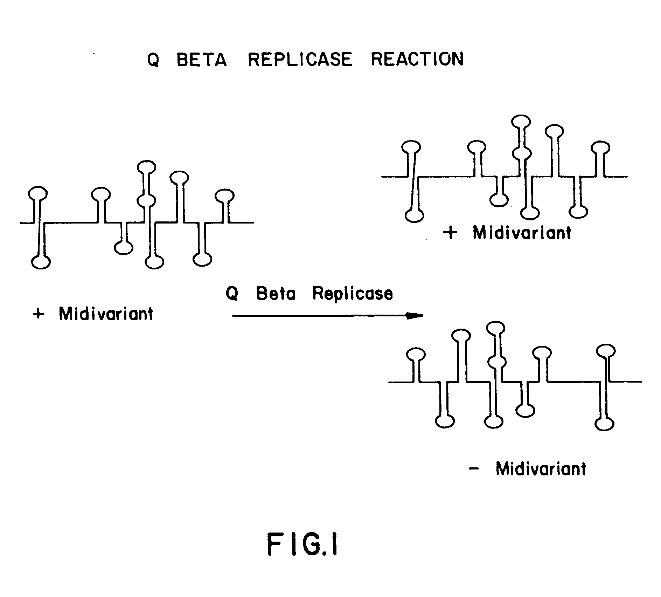

Referring to FIG. 1, the action of QB replicase is illustrated as follows: starting with a single copy of midivariant (MDV) RNA, which is symbolized by the stem and loop model of its predicted secondary structure, the strand is used as a template for the synthesis of the complementary minus strand. After N rounds of replication, there will be 2^{N} strands produced for each initial template strand. As long as the enzyme is in molar excess over the RNA strands produced, there will be an exponential production of product RNA.

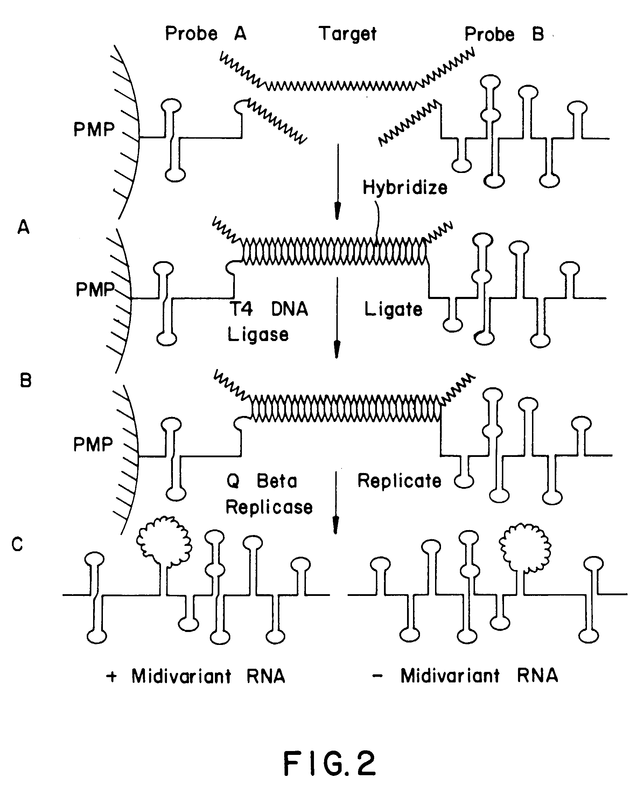

In the preferred amplification method of the present invention the midivariant (+) DNA template is divided into two nonreplicable portions for example, at base 61 from the 5′ end of the plus strand. To each of the two portions of the midivariant DNA is added a distinct target specific nucleic acid sequence which is complementary to a target nucleic acid sequence to form a conjugate. The analogous complementary sequences of the midivariant (-) DNA may be utilized in a similar manner. The two conjugates are designated Probe A and Probe B, respectively, see Fig. 2. Probe A has been immobilized upon PMP. The immobilization of a conjugate on a solid support, however, is not a requirement nor limitation of the present invention. The test sample is lysed to release the target nucleic acid sequences. The released target nucleic acid sequences are hybridized with Probe A and Probe B under hybridizing conditions. The target specific sequences are chosen so that upon hybridization to the target, the two target specific sequences are aligned contiguous to each other. After hybridization, the PMP are separated from the test sample components and unhybridized Probe B and non-targeted nucleic acid sequences.

Probes A and B which have been hybridized to the target sequence are then joined covalently by the action of DNA ligase which catalyzes the formation of a phosphodiester bond between the two Probes, see Fig. 2B. The conjugates may be released from the solid phase before or after the ligation step. Following this ligation step a midivariant (+) DNA template has been generated in a manner that depends upon the presence of the target molecule in the test sample. Inserted into this midivariant (+) DNA sequence is the combined target specific sequences from Probe A and Probe B. The midivariant template with the inserted target specific sequences can now be replicated with QB replicase. The products of the replication are detectable and thereby indicative of the presence of the target in the test sample.

For this ligation-replication method to work it is desirable that neither Probe A or Probe B alone can serve as a replicable template. In addition, the insertion of target specific sequences into the midivariant template at the position chosen must not unduly perturb those features required for its recognition by QB replicase, see Fig. 2C.

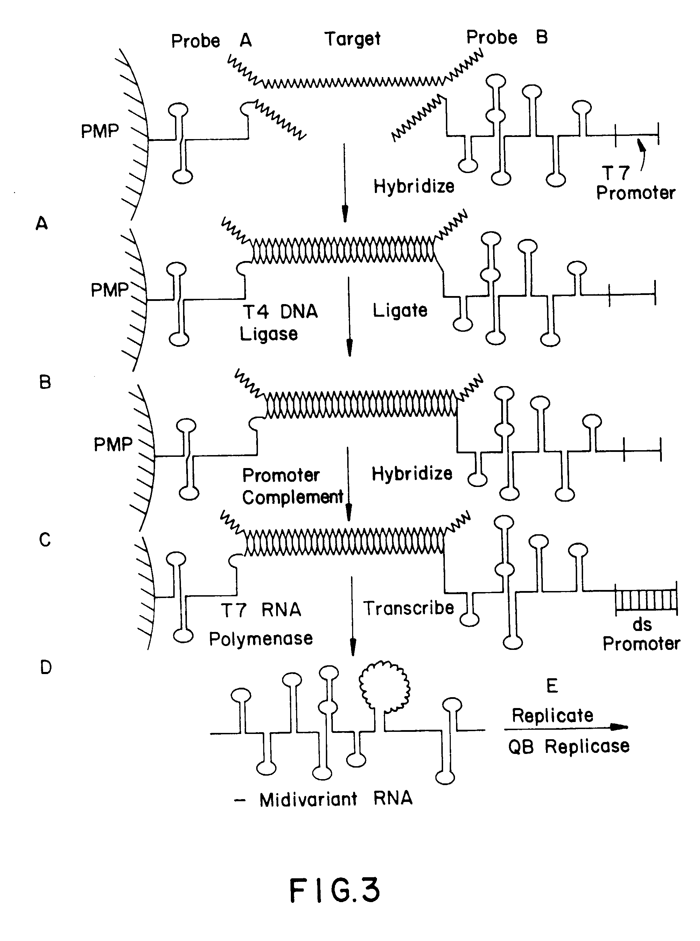

In a modification of this amplification method the ligated midivariant DNA is transcribed into RNA before the replication by QB replicase. A polymerase promoter is attached to a nonreplicable portion of midivariant DNA before the transcription step. The ligation-transcription-replication method enables the detection of a single template. See Fig. 3.

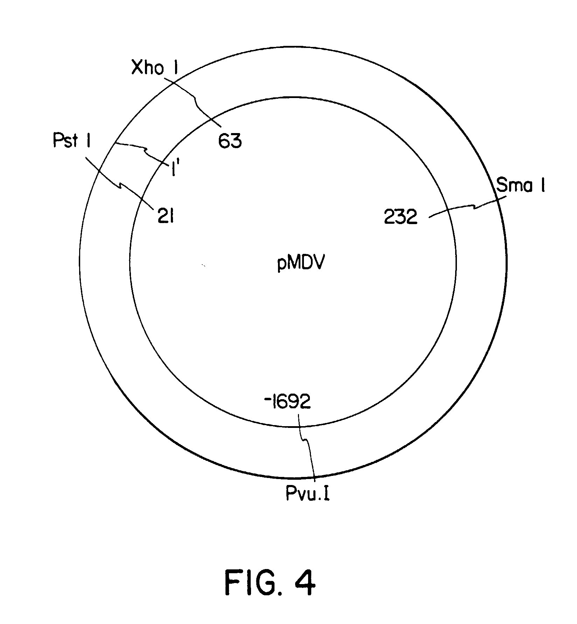

A plasmid containing the midivariant sequence (pMDV) (Promega, Madison, WI) was used to transform bacterial cells (JM107), the transformed cells grown in culture, the cultured cells harvested and lysed, and the plasmid purified, all by standard procedures. See Example 1. Samples of pMDV were digested with the restriction enzymes Pst I, Sma I, Xho I, or combinations of these enzymes to obtain the fragments of pMDV as indicated in the restriction map shown in FIG. 4. The pMDV contained a 10 base pair Xho I linker at position 61 of the plus strand.

Other versions of the midivariant sequence were prepared and tested for their capability to serve as replication templates. Advantage was taken of the Xho I restriction site in order to insert foreign target specific sequences. Insertions made into the natural midivariant sequence may be used to hybridize with desired target sequences and facilitate the use of midivariant DNA in amplification methods so long as the inserted target specific sequences permit the recombinant molecules to act as templates for RNA synthesis. Table I lists examples of inserted sequences at the Xho I restriction site.

All versions of midivariant DNA were base treated to remove possible RNA contaminants prior to inclusion in replication reactions. Intact plasmid and restriction enzyme digested plasmids were incubated at 80°C in 1 N NaOH for 15 min. and then neutralized by addition of an equivalent of HCl prior to incorporation in replicase reactions in order to remove contaminating RNA templates. A sample of each of the putative DNA template was also digested with DNase (Promega) and the digested templates also tested for residual capability to serve as a substrate for QB replicase.

Midivariant DNA samples were then tested for their ability to serve as templates for RNA synthesis with QB replicase. The replicase reactions contained 100 mM Tris-HCl, pH 7.5, 15 mM MgCl₂, 1mM each ATP, GTP, UTP, and CTP. Alpha ³²P-CTP (new England Nuclear) was utilized as a label to detect synthesized RNA products. The QB replicase concentration was 20 ug/ml and reactions incubated for 1 hr. at 37°C. The replication reaction was assayed by spotting an aliquot of the reaction on a GFF filter (Whatman), precipitating the synthesized RNA by immersing the filter in ice cold 10% trichloroacetic acid/1% sodium pyrophosphate. The filters were washed four times with ice cold 5% trichloroacetic acid and then counted by liquid scintillation. The results of these replication experiments are summarized in Table II and establish the ability of the intact midivariant DNA sequence, whether in linear or closed circular form, to act as a template for QB replicase directed RNA synthesis. It was further observed that the RNA product synthesized from midivariant DNA templates hybridized with pMDV but not to a plasmid containing the nanovariant DNA sequence (data not shown).

In order to test the sensitivity of replication of midivariant templates with inserted sequences, the sequences were cloned into an Xho site of a plasmid containing the midivariant sequence as described above. The sensitivity of replication reactions of these midivariant DNA templates are summarized in Table III. These data were obtained by measuring the incorporation of ³²P-CTP into the RNA replication product. These data indicate that the end product of the ligation-replication scheme, see FIG. 2, is a template for QB replicase and that as few as 160 template molecules (MDV-SA2) could be detected. The replication products were further characterized by Northern blotting and the inserted target specific sequences were found to be replicated along with the flanking midivariant sequences (data not shown).

It was noted that some of the QB replicase enzyme preparations utilized contained a DNase contamination. This contamination may interfere with replication sensitivity by degrading the DNA templates. The removal of the DNase contaminant by alternative purification schemes or the use DNase inhibitors may be required where contamination is present. An alternative approach to obtain better replication sensitivity is to obtain an enzyme which replicates DNA templates more efficiently than QB replicase. QB replicase itself might be modified by site-directed mutagenesis to produce a more efficient enzyme.

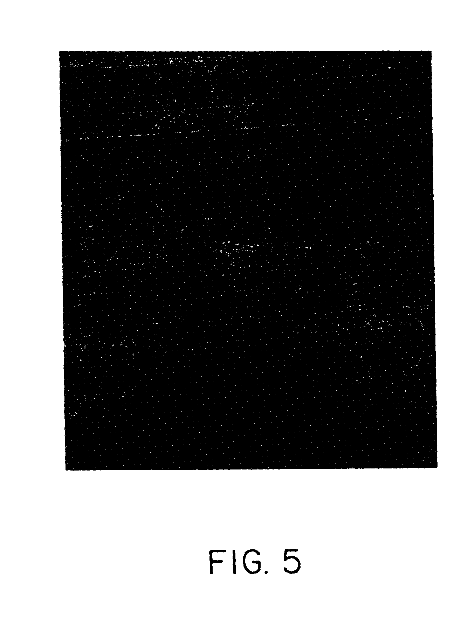

The products of the reaction of QB replicase and the various midivariant DNA templates were further characterized by denaturing polyacrylamide gel electrophoresis. Gel electrophoresis results of some of the ³²P labelled midivariant replication products are shown in FIG. 5. Midivariant DNA templates were incubated with QB replicase at 30°C for 2 hrs. Samples from each reaction were incubated in loading buffer containing 7M urea at 70°C for 5 min. prior to electrophoresis. The samples were electrophoresed on a 6% polyacrylamide gel that contained 7M urea in a Tris borate EDTA buffer. After electrophoresis the bands were visualized by autoradiography. Referring to FIG. 5 the samples applied to the lanes (left to right) are as follows: lane 1: 123 bp ladder (BRL) as a size marker; lane 2: zero template control; lane 3: replication products from pMDV template; lane 4: replication products from MDV template; lane 5: replication products from MDV-CA 116 template; lane 6: replication products from MDV-CA 29 template; and lane 7: replication products from MDV-poly template. In addition to the expected size full length replication products, for each template many additional products were observed which migrated faster than the full length products. The faster migrating bands have been shown to hybridize with probes complementary to portions of the midivariant plus strand and the minus strand (data not shown).

The replication products from the midivariant templates were further characterized by Northern blotting to determine whether the inserted sequences were replicated along with the flanking midivariant sequences. These blots were probed with a Campylobacter specific probe, PM238 (Promega) for the templates containing inserts of Campylobacter sequences or a polylinker specific probe, PM905 (Promega), see Table I. The replication products from MDV-CA 29 and MDV-CA 116 hybridized with PM238 while the replication products from MDV-poly hybridized with PM905 (data not shown). The results demonstrate that the inserted sequences replicate along with flanking midivariant sequences.

### EXAMPLE 1

### Replication Sensitivity of Midivariant DNA Templates

A plasmid containing a midivariant (pMDV) sequence and having a 10 base pair insertion to generate an Xho I linker was obtained from Promega, Inc., Madison, WI. The restriction site enables insertion of desired target specific sequences at base 61 in the plus strand of MDV DNA. Clones were prepared which consisted of the MDV plasmid containing various inserts at the Xho I site. MDV DNA templates were prepared by Pst I/Sma I digestion of the purified plasmid and purified by preparative agarose gel electrophoresis method. The concentration of the template was estimated by the intensity of the fluorescence of the ethidium bromide stained band of the purified template, analyzed by agarose gel electrophoresis. The template also contained a T7 promoter sequence that enabled the transcription of the plus strand template by T7 RNA polymerase, see FIG. 4. MDV DNA templates were base treated by incubation with 1 N NaOH at 80°C for 15 min. and then neutralized with an equivalent of HCl. This treatment degraded any RNA contamination which was present and denatured the template into single stranded DNA.

Replication reactions were carried out by incubating various amounts of MDV DNA templates, both denatured single stranded as well as native double stranded, with 20 ug/ml QB replicase (Lot M202, Promega) in 100 mM Tris-HCl, pH 7.5, 15 mM Mg Cl₂, and 1 mM each of ATP, GTP, UTP, and CTP. Alpha ³²P-CTP (Amersham) 800 Ci/mmol, was added to give a specific activity on the order of 10⁵ cpm/nmol CTP. The reactions were incubated at 30°C for 2 hrs. The incorporation of ³²P-CTP into the replication product was determined by precipitating an aliquot of the reaction solution on a glass fiber filter (Whatman) in the presence of ice cold 10% trichloroacetic acid/1% sodium pyrophosphate. The unincorporated CTP was removed by washing the filters with ice cold 5% trichloroacetic acid. The incorporated CTP was determined by liquid scintillation counting. A negative control (no added template) was included in the experiments. The results are summarized in Table III. The experiments indicate that replication sensitivity (number of templates which could be detected by replication) was greater than or equal to 500 templates for denatured, single-stranded DNA templates and less than 200 templates for the native double-stranded DNA template.

### Example 2

### Replication of Midivariant Probes

Various fragments of MDV DNA of Example 1 were tested for their ability to act as templates for replication with QB replicase. The Applied Biosystem 391 PCR-Mate DNA synthesizer was utilized for the synthesis of the oligomers. The oligomers were purified by RP-HPLC. The following oligomers were synthesized:
- MB2:: bases 120-221 of the plus strand MDV DNA
- MB1SA1:: 24 bases of Salmonella specific sequence attached to the 5' terminus of bases 62-119 of MDV (+) DNA
- MASA5:: 10 bases of non-specific sequence attached to the 5' terminus of bases 1-64 of MDV (+) DNA, 24 bases of Salmonella sequence attached to the 3' terminus
- MBSA:: MB2 and MB1SA1 ligated together (See below)
- MDSA1:: 24 bases of Salmonella specific sequence attached to the 5' terminus of bases 161-220 of MDV (-) DNA.
- MC2SA5:: 24 bases of Salmonella specific sequence attached to the 3' terminus of bases 85-160 of MDV (-) DNA.

The fragments of MDV templates prepared synthetically were tested as templates for QB replicase. The putative templates were incubated with 20 ug/ml QB replicase (Promega) (Lot M202) at 30°C for 2 hrs. in 100 mM Tris-HCl, pH 7.5, 15 mM MgCl₂, and 1 mM each ATP, GTP, UTP, and CTP. Alpha ³²P-CTP (Amersham) 800 Ci/mmol were added to give a specific activity of approximately 10⁵ cpm/nmol CTP. CTP incorporation was determined as described in Example 1. The results of these experiments are summarized in Table IV. The results indicate that at the levels examined, none of the fragments of MDV (+) or MDV (-) DNA served as templates for QB replicase.

### Example 3

### Demonstration of Hybridization-Ligation-Replication

PM2058 (Promega) a 48 base synthetic oligomer having a sequence complementary to the Salmonella portions of the probes, was incubated with 24 ug MASA5-PMP (PMP, Ciba Corning Diagnostics Corp.) and 500 fmol MBSA1 (MB2 and MB1SA1 ligated together) in 100 ul containing 4X SSC, 10 mM Tris-HCl, pH 7.5, 0.1% BSA, 0.02% Tween-20, and 5% dextran sulfate, at 56°C for 1 hr. MBSA1 was prepared by hybridization of 5' phosphorylated MB2 (10 pmol) and MB1SA1 (17 pmol) to a 33 nucleotide synthetic oligomer complementary in sequence to portions of MB2 and MB1SA1. This hybridization aligns the midivariant sequence at the 3′ terminus of MB1SA1 adjacent to the midivariant sequence at the 5′ terminus of MB2. These two oligomers were ligated together by T4 DNA ligase (Promega). The ligated product, MBSA1, was purified from the reaction mixture by preparative 6% denaturing polyacrylamide gel electrophoresis. As a zero target control, the PMP and MBSA1 were incubated alone in the absence of PM2058 under the same conditions. The PMP were separated and the supernatant removed. The PMP were washed twice with 2X SSC/0.1% Tween-20.

The PMP were resuspended in 100 ul of ligase buffer (BRL) containing 20 U T4 DNA ligase (BRL) and incubated at room temperature for 1 hr. As a control to determine the necessity of ligation, an equivalent sample of hybridized PMP were incubated in the ligase buffer without T4 DNA ligase. The PMP were separated and the supernatant removed; the PMP were then washed twice with 2X SSC/0.1% Tween-20 and resuspended in 100 ul water.

One ul aliquots from each ligation sample was incubated with 20 ug/ml QB replicase (Lot M202) (Promega) in 100 mM Tris-HCl, pH 7.5, 15 mM MgCl₂, and 1 mM each ATP, GTP, UTP, and CTP. Alpha ³²P-CTP (Amersham) 800 Ci/mmol was included to give a specific activity of approximately 10⁵ cpm/nmol CTP. CTP incorporation was determined as described in Example 1. The replication reactions were also analyzed by denaturing 6% polyacrylamide gel electrophoresis.

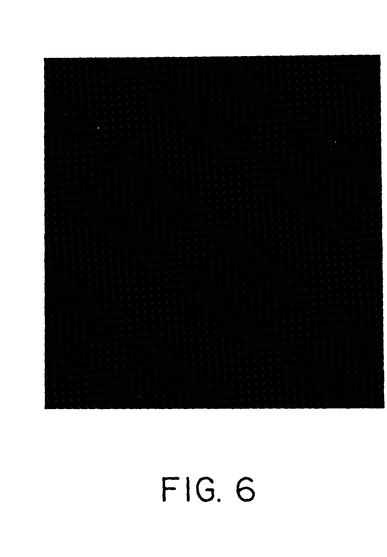

The results for CTP incorporation are summarized in Table V and the results of the gel electrophoresis are shown in FIG. 6. Referring to FIG. 6 the samples applied to the lanes (left to right) are as follows: lane 1: 123 bp ladder (BRL) as a size marker; lane 2: ligated template replication product; lane 3: Minus PM2058 Control, replication product; and lane 4: Minus ligase control, replication product. The absence of replication in the negative control sample confirms the results from Example 2 that the probes do not replicate under the conditions of the test assay.

### Example 4

### Sensitivity of Sequential Transcription-Replication

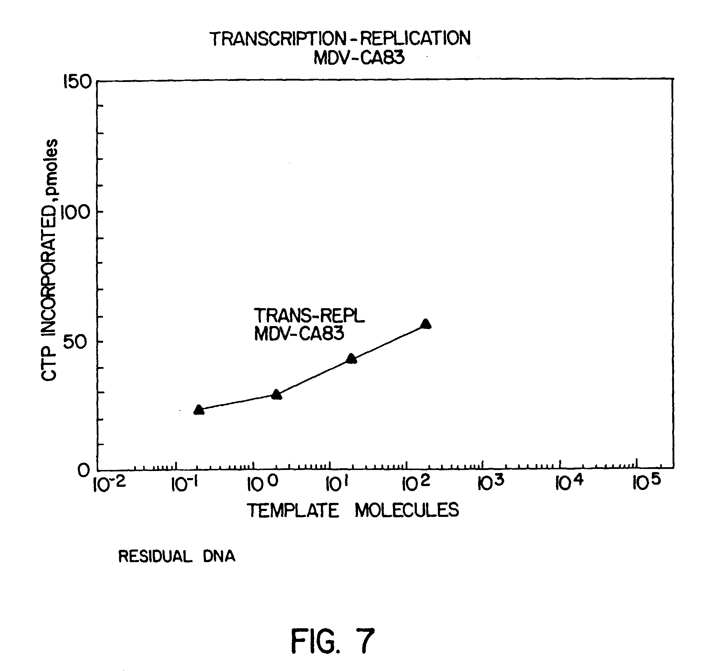

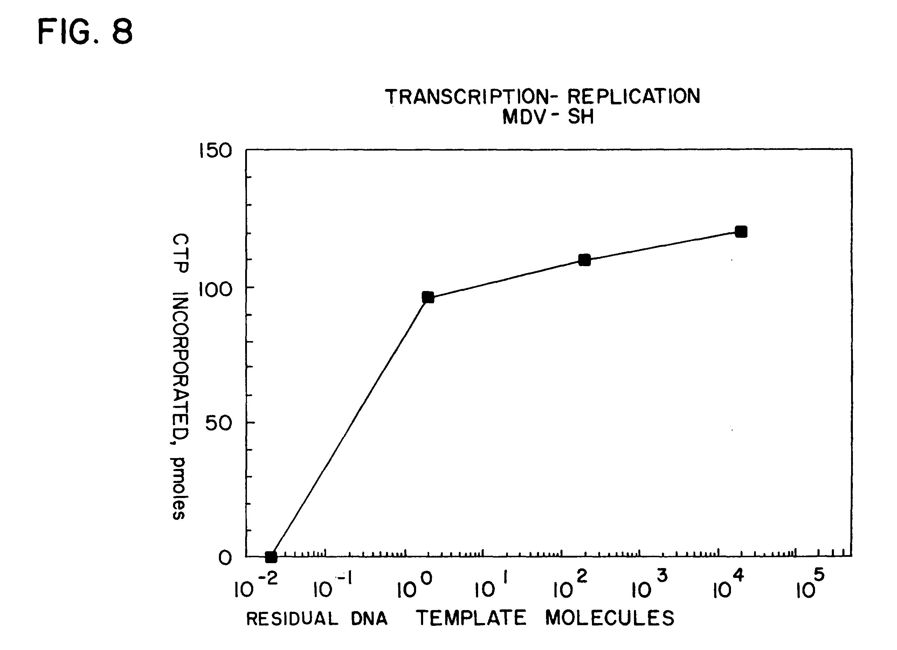

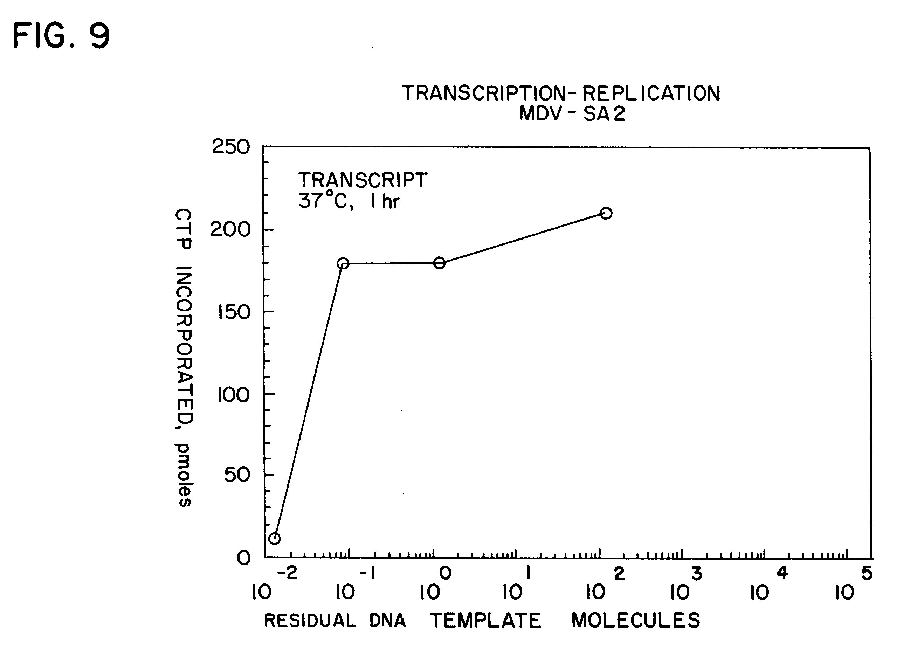

The Pst I/Sma I fragments of the midivariant plasmids containing target specific inserts including, for example, Salmonella, Shigella, and Campylobacter were prepared and gel purified. These Pst I/Sma I fragments contain a T7 promoter that enables a transcript of the plus strand of the midivariant template be be produced by the action of T7 RNA polymerase. Dilutions of the Pst I/Sma I fragments were incubated with T7 RNA polymerase (Promega) 3 U/ul for 1 hr. at 37°C in 40 mM Tris-HCl, pH 8.0, also 10 mM NaCl, 6mM MgCl₂, 2mM spermidine, 10mM dithiothrestol, and 3mM each of ATP, CTP, GTP and UTP, in a total volume of 25 ul. A 2 ul aliquot of the transcription reaction product was then incubated with QB replicase under the standard replication reaction conditions described in the preceding Examples except that the incubation was for 1 hr. at 37°C. CTP incorporation was measured as described in Example 1. The results of the transcription-replication reactions are shown in FIGs. 7, 8 and 9 for MDV-CA83, MDV-SH, and MDV-SA2, respectively. These data indicate that transcription occurred even for the lowest amount of template present and between 10 and 100 RNA copies are produced per DNA template. The RNA transcripts were efficiently replicated by QB replicase. The combined transcription-replication allowed for the detection of templates at the single molecule level.

**TABLE I**

| | |
|---|---|
| Poly: | 26 bp polylinker sequence (Promega) |
| SA1: | 48 bp Salmonella sequence (Promega) |
| SA2: | 48 bp Salmonella sequence (Promega) |
| SH: | 60 bp Shigella sequence (Promega) |
| CA: | 60 bp Campylobacter sequence (Promega) |
| CA29: | 29 bp Campylobacter 16S rRNA sequence |
| CA83: | 83 bp Campylobacter 16S rRNA sequence |
| CA116: | 116 bp Campylobacter 16S rRNA sequence |

**TABLE II**

| REPLICATION OF MIDIVARIANT PLASMID FRAGMENTS | | |
|---|---|---|
| TEMPLATE | TREATMENT | CTP INCORPORATED (pmol) |
| pMDV | Base | 720 |
| pMDV | Base/DNase | 14 |
| pMDV/Pst I | Base | 420 |
| pMDV/Pst I | Base/DNase | 10 |
| pMDV/Sma I | Base | 460 |
| pMDV/Sma I | Base/DNase | 7 |
| MDV | Base | 590 |
| MDV | Base/DNase | 22 |
| pMDV/Xho 1/Pvu I Larger Fragment | Base | 0 |
| pMDV/Xho I/Pvu I Smaller Fragment | Base | 28 |

**TABLE III**

| REPLICATION SENSITIVITY OF DNA TEMPLATES | | | |
|---|---|---|---|
| TEMPLATE | INSERT SIZE, bp | SPECIFICITY | SENSITIVITY MOLECULES |
| MDV¹, ss² | | | 1000 |
| MDV¹-SA1, ss² | 48 | Salmonella | 1500 |
| MDV¹-SA2, ds³ | 48 | Salmonella | 160 |
| MDV¹-CA, ss² | 60 | Campylobacter | 2000 |
| MDV¹-CA83, ss² | 83 | Campylobacter | 1500 |
| MDV¹-CA29, ss² | 29 | Campylobacter | 2000 |
| MDV¹-SH, ss² | 60 | Shigella | 500 |

| | | | |
|---|---|---|---|
| ¹ The template was isolated by purification of the Pst 1/Sma I fragment of the corresponding midivariant plasmid. | | | |
| ² The restriction fragment were denatured by base treatment. | | | |
| ³ This template was not base treated. | | | |

**TABLE IV**

| REPLICATION OF MIDIVARIANT FRAGMENTS | |
|---|---|
| TEMPLATE | CTP INCORPORATION, pmoles |
| MB2, 1 pmol | 0 |
| MB1SA1, 8 fmol | 0 |
| MASA5, 10 fmol | 0 |
| MB1SA1-MB2, 600 amol | 0 |
| MDSA1, 1 pmol | 0 |
| MC2SA5, 1 pmol | 0 |

**TABLE V**

| INCORPORATION OF CTP INTO REPLICATION PRODUCTS | |
|---|---|
| Sample¹ | CTP Incorporated², pmoles |
| Ligated Template | 180 |
| Minus PM2058 Control | 0 |
| Minus Ligase Control | 0 |

| | |
|---|---|
| ¹ The template (1 fmol) amount for the ligated template sample is the total amount of template in the replication reaction assuming that the hybridization and ligation steps were 100% efficient. | |
| ² The amount of CTP incorporated is for a 5 ul aliquot of the replication reaction out of a total volume of 50 ul. | |

## Claims

1. An amplification method for detecting the presence of a target nucleic acid sequence in a test sample characterised in that it comprises: hybridizing at least two midivariant DNA/probe conjugates to a target nucleic acid sequence, each-conjugate including a distinct nonreplicable portion of midivariant DNA and a distinct target specific nucleic acid sequence complementary to a target nucleic acid sequence in the test sample; ligating the conjugates to form a replicable DNA template; and replicating this template.

2. A method as claimed in claim 1 wherein it further comprises the detection of the replication product, the detection methodology preferably comprising the use of a waveguide or a radioisotopic technique or a chemiluminescent methodology.

3. A method as claimed in claim 1 or claim 2 wherein at least one conjugate includes an appended polymerase promoter, preferably a T7 RNA polymerase promoter.

4. A method as claimed in claim 3 wherein it further comprises the transcription of the midivariant DNA template to RNA prior to replication.

5. A method as claimed in any of claims 1 to 4 wherein the replication is catalyzed by QB replicase.

6. A method as claimed in any of claims 1 to 5 wherein the hybridization of the midivariant DNA/probe conjugates to the target nucleic acid sequence effects the contiguous alignment of the probes.

7. A method as claimed in any of claims 1 to 6 wherein the probes are ligated by T4 DNA ligase.

8. A method as claimed in any of claims 1 to 7 wherein at least one midivariant DNA/probe conjugate is immobilized on a solid support, preferably comprising a paramagnetic particle, the conjugate optionally being released from the solid phase before or after ligation of the probes.

9. A method as claimed in claim 8 wherein, following hybridization, the hybridized midivariant DNA/probe conjugates are separated from the unhybridized midivariant DNA/probe conjugates and other components of the test sample by magnetic separation.

10. A method as claimed in any of claims 1 to 9 wherein the nonreplicable portion of midivariant DNA comprises the first 61 nucleotides of the 5′ end of midivariant (+) DNA and the other nonreplicable portion of the midivariant DNA comprises the remaining 3′ nucleotides of midivariant (+) DNA; or wherein the nonreplicable portion of midivariant DNA comprises the first 61 nucleotides of the 3′ end of midivariant (-) DNA and wherein the other nonreplicable portion of midivariant DNA comprises the remanning 5′ nucleotides of midivariant (-) DNA.

11. A method as claimed in any of claims 1 to 10 wherein at least one conjugate further comprises a nonspecific base sequence at its 5′ terminus, the nonspecific base sequence including at least one nucleotide and wherein at least one nucleotide includes a reactive group, preferably a primary amine.

12. A method of detecting a target nucleic acid sequence in a test sample characterised in that it comprises:
(a) contacting the target nucleic acid sequence with two midivariant DNA/probe conjugates under hybridizing conditions, each conjugate comprising a distinct nucleic acid sequence complementary to the target nucleic acid sequence and a distinct nonreplicable portion of midivariant DNA, upon hybridization of the conjugates, the probes being contiguously aligned;
(b) ligating the probes, a template being formed;
(c) transcribing the DNA template to RNA;
(d) replicating the RNA transcript; and
(e) detecting the replication product of (d).

13. A conjugate characterised in that it comprises a target specific nucleic acid sequence, preferably including an oligonucleotide, which target specific nucleic acid sequence is preferably complementary to a target nucleic acid sequence in a test sample under hybridizing conditions, and a nonreplicable portion of midivariant DNA, the conjugate being a component of an amplification method as claimed in any of claims 1 to 11.

14. A conjugate pair for detecting a target nucleic acid sequence in a test sample characterised in that it comprises:
(a) a first conjugate including a target specific nucleic acid sequence and a nonreplicable portion of midivariant DNA; and
(b) a second conjugate including a target specific nucleic acid sequence and a nonreplicable portion of midivariant DNA, the target specific nucleic acid sequences of the first and second conjugates being mutually distinct and both sequences being complementary, under hybridizing conditions, to the same target to be contiguously aligned so that the target nucleic acid sequences may be ligated, and the nonreplicable portions of the midivariant DNA of the first and second conjugates being mutually distinct.

15. A test kit for detecting a target nucleic acid sequence in a test sample characterised in that it comprises:
(a) a solution for releasing target nucleic acid sequences; and
(b) at least two conjugates, at least one of which is as claimed in claim 13 or claim 14, each of the conjugates including a distinct target specific nucleic acid sequence complementary, under hybridizing conditions, to the target nucleic acid sequence; and a distinct nonreplicable portion of midivariant DNA.

## Patentansprüche

1. Amplifizierungsverfahren zum Nachweis des Vorliegens einer Target-Nukleinsäuresequenz in einer Testprobe,
dadurch gekennzeichnet,
daß es die nachfolgenden Schritte umfasst:
Hybridisierung wenigstens zweier Midivariant-DNS/Sonden-Konjungate an eine Target-Nukleinsäuresequenz, wobei jedes Konjugat einen distinkten nicht replizierbaren Anteil einer Midivariant-DNS und eine distinkte targetspezifische Nukleinsäuresequenz, die zu einer Target-Nukleinsäuresequenz in der Testprobe komplementär ist, enthält; Ligieren der Konjugate zur Bildung einer replizierbaren DNS-Matrize; und Replizieren dieser Matrize.

2. Verfahren nach Anspruch 1, weiterhin umfassend den Nachweis des Replikationsprodukts, wobei die Nachweismethode bevorzugt die Verwendung einer Wellenleiter- oder Radioisotopentechnik oder einer Chemilumineszenzmethode umfaßt.

3. Verfahren nach Anspruch 1 oder Anspruch 2, wobei wenigstens ein Konjugat einen angehängten Polymerase-Promotor, bevorzugt einen T7 RNA-Polymerase-Promotor, enthält.

4. Verfahren nach Anspruch 3, das weiterhin die Transkription der Midivariant-DNS-Matrize zu RNA vor der Replikation umfaßt.

5. Verfahren nach einem der Ansprüche 1 - 4, wobei die Replikation durch Qβ-Replicase katalysiert wird.

6. Verfahren nach einem der Ansprüche 1 - 5, wobei die Hybridisierung der Midivariant-DNS/Sonden-Konjugate an die Target-Nukleinsäuresequenz die aneinanderliegende Ausrichtung (Alignment) der Sonden bewirkt.

7. Verfahren nach einem der Ansprüche 1 - 6, wobei die Sonden durch T4 DNS-Ligase ligiert werden.

8. Verfahren nach einem der Ansprüche 1 - 7, wobei wenigstens ein Midivariant-DNS/Sonden-Konjugat auf einem Festträger, der bevorzugt ein paramagnetisches Teilchen umfaßt, immobilisiert wird, wobei das Konjugat wahlweise vor oder nach der Ligierung der Sonden von der Festphase freigesetzt wird.

9. Verfahren nach Anspruch 8, wobei nach der Hybridisierung die hybridisierten Midivariant-DNS/Sonden-Konjugate von den nicht hybridisierten Midivariant-DNS/Sonden-Konjugaten und anderen Bestandteilen der Testprobe durch magnetische Abtrennung abgetrennt werden.

10. Verfahren nach einem der Ansprüche 1 - 9, wobei der nicht replizierbare Anteil der Midivariant-DNS die ersten 61 Nukleotide des 5'-Endes der Midivariant-(+)-DNS und der andere, nicht replizierbare Anteil der Midivariant-DNS die verbleibenden 3'-Nukleotide der Midivariant-(+)-DNS umfaßt; oder wobei der nicht replizierbare Anteil der Midivariant-DNS die ersten 61 Nukleotide des 3'-Endes der Midivariant-(-)-DNS umfaßt und wobei der andere, nicht replizierbare Anteil der Midivariant-DNS die verbleibenden 5'-Nukleotide der Midivariant-(-)-DNS umfaßt.

11. Verfahren nach einem der Ansprüche 1 - 10, wobei wenigstens ein Konjugat weiterhin eine nicht spezifische Basensequenz an seinem 5'-Terminus umfaßt, wobei die nicht spezifische Basensequenz wenigstens ein Nukleotid enthält und wobei wenigstens ein Nukleotid eine reaktive Gruppe, bevorzugt ein primäres Amin, enthält.

12. Verfahren zum Nachweis einer Target-Nukleinsäuresequenz in einer Testprobe, dadurch gekennzeichnet, daß es die nachfolgenden Schritte umfaßt:
(a) in Kontakt bringen der Target-Nukleinsäuresequenz mit zwei Midivariant-DNS/Sonden-Konjugaten unter Hybridisierungsbedingungen, wobei jedes Konjugat eine distinkte, zur Target-Nukleinsäuresequenz komplementäre Nukleinsäuresequenz und einen distinkten, nicht replizierbaren Anteil der Midivariant-DNS umfaßt, wobei bei Hybridisierung der Konjugate die Sonden aneinanderliegend ausgerichtet werden;
(b) Ligieren der Sonden unter Bildung einer Matrize;
(c) Transkribieren der DNS-Matrize in RNA;
(d) Replizieren des RNA-Transkripts; und
(e) Nachweis des Replikationsprodukts von (d).

13. Konjugat,
dadurch gekennzeichnet,
daß es eine targetspezifische Nukleinsäuresequenz, bevorzugt mit einem Oligonukleotid, wobei die targetspezifische Nukleinsäuresequenz bevorzugt zu einer Target-Nukleinsäuresequenz in einer Testprobe unter Hybridisierungsbedingungen komplementär ist, und einen nicht replizierbaren Anteil der Midivariant-DNS umfaßt, wobei das Konjugat ein Bestandteil eines Amplifizierungsverfahrens nach einem der Ansprüche 1 - 11 ist.

14. Konjugatpaar zum Nachweis einer Target-Nukleinsäuresequenz in einer Testprobe,
dadurch gekennzeichnet,
daß es umfaßt:
(a) ein erstes Konjugat mit einer targetspezifischen Nukleinsäuresequenz und einem nicht replizierbaren Anteil der Midivariant-DNS; und
(b) ein zweites Konjugat mit einer targetspezifischen Nukleinsäuresequenz und einem nicht replizierbaren Anteil der Midivariant-DNS, wobei die targetspezifischen Nuleinsäuresequenzen der ersten und zweiten Konjugate wechselseitig distinkt und beide Sequenzen zu dem gleichen, aneinanderliegend anzuordnenden Target unter Hybridisierungsbedingungen komplementär sind, so daß die Target-Nukleinsäuresequenzen ligierbar sind, und die nicht replizierbaren Anteile der Midivariant-DNS der ersten und zweiten Konjugate wechselseitig distinkt sind.

15. Testkit zum Nachweis einer Target-Nukleinsäuresequenz in einer Testprobe,
dadurch gekennzeichnet,
daß er umfaßt:
(a) eine Lösung zur Freisetzung der Target-Nukleinsäuresequenz und
(b) zumindest zwei Konjugate, von denen zumindest eines wie in Anspruch 13 oder Anspruch 14 beansprucht ist, wobei beide Konjugate eine distinkte targetspezifische Nukleinsäuresequenz aufweisen, die unter Hybridisierungsbedingungen zur Target-Nukleinsäuresequenz komplementär sind; und einen distinkten, nicht replizierbaren Anteil der Midivariant-DNS.

## Revendications

1. Une méthode d'amplification pour détecter la présence d'une séquence cible d'acide nucléique dans un échantillon à tester, caractérisée en ce qu'elle comprend : l'hybridation d'au moins deux conjugués d'ADN midivariant/sonde à une séquence cible d'acide nucléique, chaque conjugué comprenant une portion distincte non réplicable d'ADN midivariant et une séquence distincte d'acide nucléique spécifique de cible qui est complémentaire d'une séquence cible d'acide nucléique dans l'échantillon à tester ; la ligature des conjugués pour former une matrice d'ADN réplicable ; et la réplication de cette matrice.

2. Une méthode telle que revendiquée dans la revendication 1, qui comprend de plus la détection du produit de la réplication, la méthodologie de détection comprenant de préférence l'utilisation d'un guide d'ondes ou une technique radio-isotopique ou une méthodologie par chimiluminescence.

3. Une méthode telle que revendiquée dans la revendication 1 ou la revendication 2, dans laquelle au moins un conjugué comprend un promoteur de polymérase attaché, de préférence un promoteur d'ARN-polymérase de T7.

4. Une méthode telle que revendiquée dans la revendication 3, qui comprend de plus la transcription en ARN de la matrice d'ADN midivariant avant la réplication.

5. Une méthode telle que revendiquée dans l'une quelconque des revendications 1 à 4, dans laquelle la réplication est catalysée par la réplicase de QB.

6. Une méthode telle que revendiquée dans l'une quelconque des revendications 1 à 5, dans laquelle l'hybridation des conjugués d'ADN midivariant/sonde à la séquence cible d'acide nucléique réalise l'alignement des sondes en contiguïté.

7. Une méthode telle que revendiquée dans l'une quelconque des revendications 1 à 6, dans laquelle les sondes sont ligaturées par l'ADN-ligase de T4.

8. Une méthode telle que revendiquée dans l'une quelconque des revendications 1 à 7, dans laquelle au moins un conjugué d'ADN midivariant/sonde est fixé sur un support solide, comprenant de préférence une particule paramagnétique, le conjugué étant facultativement libéré de la phase solide avant ou après la ligature des sondes.

9. Une méthode telle que revendiquée dans la revendication 8, dans laquelle, après l'hybridation, les conjugués d'ADN midivariant/sonde hybridés sont séparés des conjugués d'ADN midivariant/sonde non hybridés et des autres constituants de l'échantillon à tester, par séparation magnétique.

10. Une méthode telle que revendiquée dans l'une quelconque des revendications 1 à 9, dans laquelle la portion non réplicable de l'ADN midivariant comprend les 61 premiers nucléotides de l'extrémité 5' de l'ADN midivariant (+) et l'autre portion non réplicable de l'ADN midivariant comprend les nucléotides 3' restants de l'ADN midivariant (+) ; ou dans laquelle la portion non réplicable de l'ADN midivariant comprend les 61 premiers nucléotides de l'extrémité 3' de l'ADN midivariant (-) et l'autre portion non réplicable de l'ADN midivariant comprend les nucléotides 5' restants de l'ADN midivariant (-).

11. Une méthode telle que revendiquée dans l'une quelconque des revendications 1 à 10, dans laquelle au moins un conjugué comprend de plus une séquence de bases non spécifique à son extrémité 5', la séquence de bases non spécifique comprenant au moins un nucléotide et dans laquelle au moins un nucléotide comprend un groupe réactif, de préférence une amine primaire.

12. Une méthode de détection d'une séquence cible d'acide nucléique dans un échantillon à tester, caractérisée en ce qu'elle consiste à :
(a) mettre en contact la séquence cible d'acide nucléique avec deux conjugués d'ADN midivariant/sonde dans des conditions d'hybridation, chaque conjugué comprenant une séquence distincte d'acide nucléique complémentaire de la séquence cible d'acide nucléique et une portion distincte non réplicable d'ADN midivariant, les sondes étant alignées en contiguïté lors de l'hybridation des conjugués ;
(b) ligaturer les sondes, une matrice étant formée ;
(c) transcrire en ARN la matrice d'ADN ;
(d) répliquer l'ARN transcrit ; et
(e) détecter le produit de réplication de (d).

13. Un conjugué caractérisé en ce qu'il comprend une séquence d'acide nucléique spécifique de cible, comprenant de préférence un oligonucléotide, laquelle séquence d'acide nucléique spécifique de cible est de préférence complémentaire d'une séquence cible d'acide nucléique dans un échantillon à tester dans des conditions d'hybridation, et une portion non réplicable d'ADN midivariant, le conjugué étant un composant d'une méthode d'amplification telle que revendiquée dans l'une quelconque des revendications 1 à 11.

14. Une paire de conjugués servant à détecter une séquence cible d'acide nucléique dans un échantillon à tester, caractérisée en ce qu'elle comprend :
(a) un premier conjugué comprenant une séquence d'acide nucléique spécifique de cible et une portion non réplicable d'ADN midivariant ; et
(b) un second conjugué comprenant une séquence d'acide nucléique spécifique de cible et une portion non réplicable d'ADN midivariant, les séquences d'acide nucléique spécifiques de cible des premier et second conjugués étant distinctes l'une de l'autre et les deux séquences étant complémentaires de la même cible, dans des conditions d'hybridation, de façon à être alignées en contiguïté pour que les séquences d'acide nucléique spécifiques de cible puissent être ligaturées, et les portions non réplicables de l'ADN midivariant des premier et second conjugués étant distinctes l'une de l'autre.

15. Un nécessaire de test pour détecter une séquence cible d'acide nucléique dans un échantillon à tester, caractérisé en ce qu'il comprend :
(a) une solution pour libérer les séquences cibles d'acide nucléique ; et
(b) au moins deux conjugués, dont l'un au moins est tel que revendiqué dans la revendication 13 ou la revendication 14, chacun des conjugués comprenant une séquence distincte d'acide nucléique spécifique de cible qui est complémentaire, dans des conditions d'hybridation, de la séquence cible d'acide nucléique ; et une portion distincte non réplicable d'ADN midivariant.
